# EUROPEAN PATENT APPLICATION

(11) **EP 4 417 239 A1**
(43) Date of publication of application: **21.08.2024**
(21) Application number: 24153481.7
(22) Date of filing: 23.01.2024
(51) Int. Cl.: A61M 21/00, A61B 5/00, B64D 11/00, G16H 20/00

(54) **SYSTEM AND METHOD FOR REMOTELY WAKING A RESTING PERSON**

(30) Priority: 17.02.2023 IN 202311010759; 06.04.2023 US 202318296855
(71) Applicant: Honeywell International Inc., Charlotte, NC 28202 (US)
(72) Inventor: RAVINDRANATH, Balasubramanyam, Charlotte, 28202 (US); MARCHE, Stephane, Charlotte, 28202 (US); BALASA, Swetha, Charlotte, 28202 (US)
(74) Representative: Lucas, Peter Lawrence

(57) **Abstract**

A system and method of waking a person includes receiving, in a processing system, stimuli configuration data that is indicative of all available waking stimuli available for use. Sleep state data that is indicative of a current sleep state of the person is received in the processing system. The stimuli configuration data and the sleep state data are processed, in the processing system, to determine (i) a set of the available stimuli to use to awaken the person and (ii) a sequence of activation of the set available stimuli to use. The set of available stimuli are selectively activated, via the processing system, in the determined sequence of activation.

## Description

### CROSS-REFERENCE TO RELATED APPLICATION

The present application claims benefit of prior filed India Provisional Patent Application No. 202311010759, filed February 17, 2023, which is hereby incorporated by reference herein in its entirety.

### TECHNICAL FIELD

The present invention generally relates to on-duty/off-duty work alternation planning, and more particularly relates to systems and methods for remotely waking an off-duty, resting person.

### BACKGROUND

There are many working environments in which teams of coworkers alternate between on-duty status (actively performing tasks) and off-duty status (resting and/or sleeping). For example, flight crews on long-haul flights, flight crews performing several shorter flights per day, truck drivers, ship crews, medical personnel, emergency response services, surveillance teams (e.g., power plants, border control, and security), firefighters, and military personnel are subject to such alternating work cycles.

In the aviation context, and more specifically for long-haul flights (e.g., 9 or more hours), there are typically multiple pilots onboard the aircraft - one or two pilots flying and one pilot resting. However, some flights may include up to four pilots. Nonetheless, economics is driving many airlines to contemplate reducing the number of pilots for long-haul flights by implementing Reduced Crew Operations and/or extended Minimum Crew Operations (eMCO), in which the airlines allocate only two pilots for the entire flight duration for flights that currently require three pilots, and only three pilots for the entire flight duration for flights that currently require four pilots. Specifically, during the departure and arrival phases, both pilots are on-duty, implementing their normal responsibilities. However, during the cruise phase, the aircraft is flown by only one pilot (the on-duty pilot) and the other pilot(s) (the off-duty pilot) is resting/sleeping.

The two pilots may take turns flying the aircraft during the cruise phase based on a predetermined schedule. For example, during a long-haul flight that lasts about 16 hours, a pilot may fly for about 2 hours and sleep for about another 2 hours. In such a scenario, each pilot gets about 8 hours of flying time and about 8 hours of resting/sleeping time. The resting pilot would be woken up at the end of the resting schedule. But when there are emergencies (systems failure, on duty pilot incapacitation, or high workload during adverse environmental conditions such as rough weather) during flight, it is imperative for the resting pilot to be recalled to the cockpit immediately even if the resting pilot did not complete the resting period. Also, the manner in which the resting pilot is awakened (abruptly or otherwise) may affect his/her cognitive abilities throughout the rest of the flight/day.

Hence, there is a need for a system and method for waking up a resting person, such as an off-duty pilot, that is effective and done in a manner in which his/her cognitive abilities are not diminished.

### BRIEF SUMMARY

This summary is provided to describe various concepts in a simplified form that are further described in the Detailed Description. This summary is not intended to identify key or essential features of the claimed subject matter, nor is it intended to be used as an aid in determining the scope of the claimed subject matter.

In one embodiment, a system for waking a resting person includes a processing system. The processing system is configured to receive stimuli configuration data and sleep state data and is configured, upon receipt of these data, to: determine (i) a set of the available stimuli to use to awaken the person and (ii) a sequence of activation of the set available stimuli to use; and selectively activate the set of available stimuli in the determined sequence of activation. The stimuli configuration data is indicative of all available waking stimuli available for use, and the sleep state data is indicative of a current sleep state of the person.

In another embodiment, a method of waking a person includes receiving, in a processing system, stimuli configuration data that is indicative of all available waking stimuli available for use. Sleep state data that is indicative of a current sleep state of the person is received in the processing system. The stimuli configuration data and the sleep state data are processed, in the processing system, to determine (i) a set of the available stimuli to use to awaken the person and (ii) a sequence of activation of the set available stimuli to use. The set of available stimuli are selectively activated, via the processing system, in the determined sequence of activation.

Furthermore, other desirable features and characteristics of the system and method will become apparent from the subsequent detailed description and the appended claims, taken in conjunction with the accompanying drawings and the preceding background.

### BRIEF DESCRIPTION OF DRAWINGS

The present disclosure will hereinafter be described in conjunction with the following drawing figures, wherein like numerals denote like elements, and wherein:
FIG. 1 depicts a functional block diagram of one embodiment of a system for waking a resting/off-duty person; and
FIG. 2 depicts a process, in flowchart form, that may be implemented by the system of FIG. 1.

### DETAILED DESCRIPTION

The following detailed description is merely exemplary in nature and is not intended to limit the invention or the application and uses of the invention. As used herein, the word "exemplary" means "serving as an example, instance, or illustration." Thus, any embodiment described herein as "exemplary" is not necessarily to be construed as preferred or advantageous over other embodiments. All of the embodiments described herein are exemplary embodiments provided to enable persons skilled in the art to make or use the invention and not to limit the scope of the invention which is defined by the claims. Furthermore, there is no intention to be bound by any expressed or implied theory presented in the preceding technical field, background, brief summary, or the following detailed description.

With the above in mind, although the description below may refer, at least at some portions, to an aircraft environment, the system and method described herein may be used in numerous and varied environments including, but not limited to, truck drivers, ship crews, medical personnel, emergency response services, surveillance teams (e.g., power plants, border control, and security), firefighters, and military personnel, both in a vehicular context and a stationary, non-vehicular context.

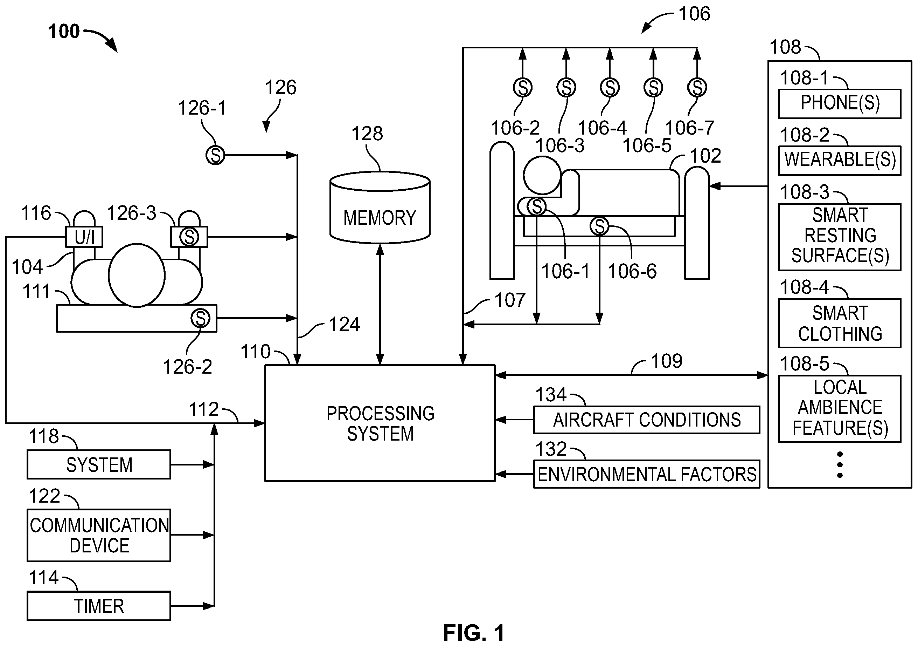

Referring to FIG. 1, a functional block diagram of one embodiment of system 100 for waking a resting/off-duty person 102 so that the resting person 102 may take over the responsibilities of a second/on-duty person 104. The depicted system 100 includes at least one or more first sensors 106, a plurality of waking stimulus sources 108, and a processing system 110. The one or more first sensors 106 are disposed on, or at least adjacent to, the resting person 102. The one or more first sensors 106 are each configured to supply sleep state data that is indicative of the current sleep state of the resting person 102. It will be appreciated that the system 100 depicted in FIG. 1 may be implemented wholly or partially in any one of numerous existing systems. For example, in the context of an aircraft, the system 100 may be implemented wholly or partially in an avionics system.

Before proceeding further, it is noted that the term sleep state generally refers to the physiological state of a person that is characterized by minimal functional and metabolic activities (both physical and mental). This term, as used herein, also refers to a numerical value or a multidimensional parameter that corresponds to a measure of the sleep state the resting person 102. Based on the sleep state data, the sleep state of the resting person 102 may be categorized as, for example, being in N1, N2, N3, or REM sleep levels, and in any of these sleep levels, the sleep state may be further categorized as disturbed (e.g., excessive body movement), and/or whether the resting person 102 may be further categorized as fatigued, just to name a few non-limiting examples of sleep state categories. The sleep state data may also indicate that the resting person 102 is incapacitated.

Returning now to the description, it will be appreciated that the number and type of the one or more first sensors 106 may vary. For example, the one or more first sensors 106 may include one or more of a smart watch, a video sensor, one or more electroencephalogram (EEG) sensors, one or more electrooculogram (EOG) sensors, one or more inertial sensors, one or more rest surface sensors, an electrocardiogram (ECG) sensor, and/or a breathing sensor, just to name a few.

The smart watch 106-1, if included, may be implemented using any one of numerous known wearable watches that are configured to implement sleep tracker technology and provide sleep state data. The video sensor 106-2, if included, may be disposed adjacent to the resting person 102 and detect, for example, various bodily movements of the resting person 102. The one or more EEG sensors 106-3 sensors, if included, are disposed on the resting person 102 and detect, for example, the brain wave activity of the resting person's brain. The one or more EOG sensors 106-4, when included, are also disposed on the second/on-duty person 104 and detect, for example, eye movements of the resting person 102. The one or more inertial sensors 106-5, when included, are disposed on the resting person 102 and detect, for example, bodily movements of the resting person 102. The one or more rest surface load sensors 106-6, when included, are disposed within the one or more resting surfaces, such as a pillow, a bed, and/or a seat, and detect, for example, movements of the resting person 102. The one or more ECG sensors 106-7, when included, are disposed on the resting person 102 and detect the heart rate of the resting person 102.

It is known that brain wave activity, eye movement, and bodily movement can all be used to measure a person's sleep duration, sleep phase, and time in (or since) a particular sleep phase. These factors, as is also known, can be correlated to, and used to provide a measure of, the sleep state of a person, such as the resting person 102. The one or more sensors 106 may also simply detect when the resting person 102 goes into a resting position and when they come out of the resting position. In between these two events, it could be assumed that the resting person 102 is sleeping. The system 100 may also, in some embodiments, include a user interface, either on the smart watch 106-1 or separately disposed, that, upon input from the resting person 102, supplies a signal to the processing system 110 indicating that they have awakened.

As may be appreciated, disposing a plurality of sensors, such as the one or more first sensors 106, on a person can potentially cause discomfort and adversely impact the person's rest state. Thus, in some embodiments, some or all of the one or more first sensors 106 may be disposed on or within a wearable textile.

The waking stimulus sources 108 are also disposed on, or adjacent to, the resting person 102. The waking stimulus sources 108 are each configured to supply stimuli configuration data indicating that it is available for use and, when activated, to generate and supply a waking stimulus to the resting person 102. It will be appreciated that the waking stimulus provided by each waking stimulus source 108 may vary, depending on how it is specifically implemented, but may include one or more of a visual, an aural, and/or a vibrational stimulus. Some non-limiting examples of suitable waking stimulus sources 108 include one or more phones 108-1, which may be a mobile phone and/or a permanently installed phone, such as a Crew Rest Area phone, a cockpit phone, and/or a cabin phone (which can supply visual and/or aural and/or vibrational stimuli), one or more wearables 108-2, such as one or more earphones (which may supply aural and/or vibrational stimuli) and/or one or more wristbands (which may supply visual and/or aural and/or vibrational stimuli), one or more smart resting surfaces 108-3, such as a smart pillow, a smart bed, and/or a smart seat (which can provide aural and/or vibrational stimuli), smart clothing 108-4 (which may be implemented using the above mentioned wearable textile) having trigger points in the fabric (which can provide vibrational stimuli), and various other permanently installed local ambience features 108-5, such as speakers, lighting, and/or temperature controls.

No matter the number and how the one or more first sensors 106 and/or the one or more waking stimulus sources 108 are specifically implemented, each is in operable communication with the processing system 110. The processing system 110 may include one or more processors and computer-readable storage devices or media encoded with programming instructions for configuring the processing system 110. The one or more processors may be any custom-made or commercially available processor, a central processing unit (CPU), a graphics processing unit (GPU), an application specific integrated circuit (ASIC), a field programmable gate array (FPGA), an auxiliary processor among several processors associated with the controller, a semiconductor-based microprocessor (in the form of a microchip or chip set), any combination thereof, or generally any device for executing instructions.

The computer readable storage devices or media may include volatile and nonvolatile storage in read-only memory (ROM), random-access memory (RAM), and keep-alive memory (KAM), for example. KAM is a persistent or non-volatile memory that may be used to store various operating variables while the processor is powered down. The computer-readable storage device or media may be implemented using any of a number of known memory devices such as PROMs (programmable read-only memory), EPROMs (electrically PROM), EEPROMs (electrically erasable PROM), flash memory, or any other electric, magnetic, optical, or combination memory devices capable of storing data, some of which represent executable programming instructions, used by the one or more processors.

The processing system 110 is coupled to receive the stimuli configuration data 109 from the one or more waking stimulus sources 108 and the sleep state data 107 from the one or more first sensors 106. The processing system 108 is configured, upon receipt of these data, to determine the set of the available waking stimuli 108 that may be used to awaken the resting person 102, and the sequence in which the set of available stimuli 108 is to be activated. Upon making this determination, the processing system 110 is configured to selectively activate the set of available waking stimuli sources 108 and do so in the determined activation sequence.

The processing system 110 is preferably configured to initiate the waking stimuli activation in response to a trigger. More specifically, and as FIG. 1 further depicts, the processing system 110 is further coupled to receive a trigger signal 112 and is configured, upon receipt of the trigger signal 112, to selectively activate the set of available stimuli in the determined sequence of activation. The trigger signal 112 may be supplied from any one of numerous sources. For example, the trigger signal 112 may be supplied from a timer 114, which may be implemented as part of the processing system 110 or separate from the processing system 110. In either case, the timer 114 is configured to generate and supply the trigger signal 112 when the resting person 102 has been off-duty for a predetermined amount of time as set, for example, by a predetermined on-duty/off-duty schedule.

The trigger signal 112 may also be supplied from one or more other sources. For example, the trigger signal 112 at least in some embodiments, may also (or instead), be supplied from one or more of a wearable user interface 116 disposed on the second/on-duty person 104, from a second processing system 118, such as another avionic system, and/or a communication device 122, such as a phone, an uplink call/message from the ground, or a side-link call/message from another aircraft.

The processing system 110 may also, at least in some embodiments, initiate the waking stimuli activation based on the cognitive state of the second/on-duty person 104. In these embodiments, and as FIG. 1 further depicts, the processing system 110 is further coupled to receive on-duty person state data 124, which is indicative of the cognitive state of the second/on-duty person 104. The processing system 110 is further configured, upon receipt of the on-duty person state data 124, to determine the cognitive state of the second/on-duty person 104. Based on the determined cognitive state of the second/on-duty person 104, the processing system 110 may itself selectively generate the trigger that initiate the waking stimuli activation.

Before proceeding further, it is noted that the term cognitive state generally refers the physiological state of a person that is characterized by reduced mental or physical performance capability that can impair the person's alertness and ability to operate a system or perform duties. This term, as used herein, also refers to a numerical value or a multidimensional parameter that corresponds to a measure of the cognitive state of the second/on-duty person 104. Based on the on-duty person state data 124, the cognitive state of the second/on-duty person 104 may be categorized as, for example, being bored, being fatigued, and/or indicate that the second/on-duty person 104 is incapacitated.

The on-duty person state data 124 may be supplied from, for example, a second set of sensors 126. The second set of sensors 126 preferably includes one or more second sensors (e.g., 126-1, 126-2, 126-3) that are disposed on, or at least adjacent to, the second/on-duty person 104. The one or more second sensors 126 are each configured to supply the on-duty person state data 124. It will be appreciated that the number and type of sensors that comprise the second set of sensors 126 may vary. Some non-limiting examples of suitable sensors includes one or more video sensors 126-1, one or more seat load sensors 126-2, and one or more electrocardiogram (ECG) sensors 126-3. In other embodiments, the first set of sensors 126 may also include inertial sensors, electro-dermal sensors, and eye tracking sensors, just to name a few non-limiting examples.

The one or more video sensors 126-1 are disposed adjacent to the second/on-duty person 104 and detect, for example, various facial features and bodily movements of the second/on-duty person 104. These facial features may vary, but include, for example, one or more of eyelid movements, eye movements, gaze direction, mouth morphology, and facial expressions, just to name a few. The one or more seat load sensors 126-2 are disposed within the second/on-duty person's seat and detect, for example, the posture state and in-seat movements of the second/on-duty person 104. The one or more ECG sensors 126-3 are disposed on the second/on-duty person 104 and detect the heart rate of the second/on-duty person 104. It is known that various facial features, body and facial movements, posture, in-seat activity, and heart rate can all be correlated to, and used to provide a measure of, the cognitive state of a person, such as the second/on-duty person 104.

In addition to determining when to initiate the waking stimuli activation in the determined activation sequence, the processing system 110 is further configured to determine the activation method to be used for activating the set of available stimuli 108. The activation methods may vary and may include, for example, a gradual increase in the intensity of the activated stimuli, an abrupt activation at a first predetermined intensity, an abrupt activation at a second predetermined intensity that is greater in magnitude than the first predetermined intensity, and multiple abrupt activations at the first or second predetermined intensity. The processing system 110 may also be configured to simultaneously activate two or more waking stimuli 108, using the determined activation method, when needed.

The number and type of waking stimuli 108 to activate, and which activation method(s) to use, may vary based on the response of the resting person 102 to the waking stimuli 108, both in real-time and from a previous history of the resting person 102. To determine the real-time response of the resting person 102, the processing system 110 is further configured, in response to the sleep state data 107, to monitor the response of the resting person 102 to each of the set of available stimuli 108 that is activated and determine the effectiveness of the activated stimuli. To determine the response of the resting person 102 from a previous history, the system 100 may additionally include a memory 128 that is in operable communication with the processing system 110 and that has user profile data stored therein that is associated with the resting person 102. The processing system 110 is further configured to selectively update the user profile data to reflect the response of the person to each set of available stimuli 108 that is activated.

In addition to taking into account the sleep state of the resting person 102, the processing system 110 may also, in some embodiments, receive data indicative of, and thus take into account, various environmental factors (e.g., noise level, lighting level, temperature, etc.) of the resting person's rest environment 132 to determine the number and type of waking stimuli 108 to activate, and which activation method(s) to use. The processing system 110 may also, in some embodiments, receive data indicative of, and thus take into account, aircraft conditions (e.g., nominal workload, high workload, turbulence, emergency, etc.) to determined number and type of waking stimuli 108 to activate, and which activation method(s) to use.

No matter the specific type of data supplied to, and processed by, the processing system 110, the main objective is to determine the effectiveness of each waking stimuli 108 in waking up the resting person 102, which is typically based on one or more of the various factors described above, and to build a person-specific profile for the resting person 102. The person-specific profile includes person-specific data and can be implemented using a predefined table, or the person-specific profile can be built using machine-learning, or it can be built using a hybrid of these two methods.

The person-specific profile may additionally include data indicative of specific characteristics of the waking stimuli that are more effective in waking the resting person 102. This data may include, for example, favorite tones (music, ringtone, pet dog barking, family member voice), preferred language, most effective actuator, and most effective actuation method, just to name a few. The person-specific profile may additionally include data indicative of the time taken to wakeup, the time taken to reorient, and whether the resting person 102 subsequently performed duties in an optimal manner after wakeup.

It should be noted that, at least in some embodiments, the person-specific profile data is preferably stored cryptographically such that the data can only be accessed when the specific person provides their consent.

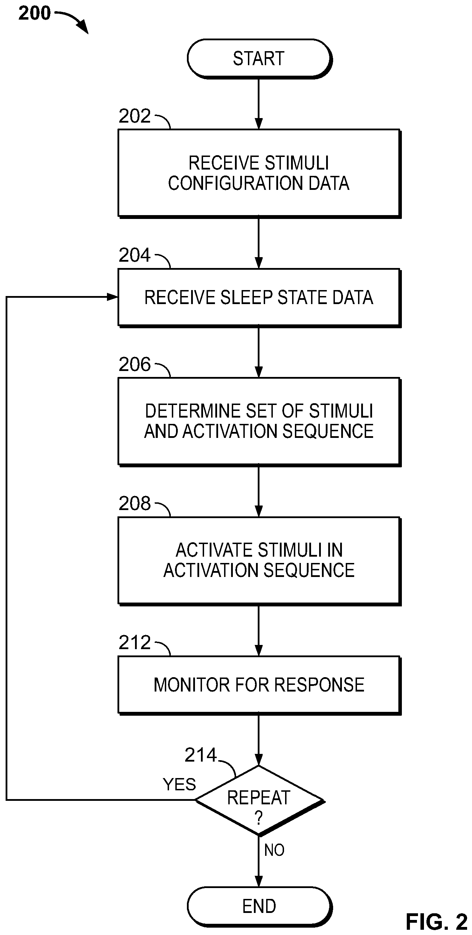

Referring now to FIG. 2, a process flowchart is depicted of one example process 200 for waking a person. The order of operation within the process 200 is not limited to the sequential execution as illustrated in the figure, but may be performed in one or more varying orders as applicable and in accordance with the present disclosure.

The example process 200, which may be implemented in the processing system 110, includes receiving the stimuli configuration data (202) and the sleep state data (204). As noted above, the stimuli configuration data is indicative of all of the waking stimuli 108 that are available for use, and the sleep state data is indicative of the current sleep state of the resting person 102. As was also noted above, the processing system 110 may also be supplied with data indicative of various environmental factors. The processing system then processes the stimuli configuration data and the sleep state data (and environmental data, if included) to determine the set of the available stimuli to use to awaken the person and the sequence in which to activate the set available stimuli (206). The processing system 110 then selectively activates the set of available stimuli in the determined sequence of activation (208).

The process 200 depicted in FIG. 2 also depicts some additional process steps that may be implemented such as, for example, monitoring for a response to the stimuli (212) and evaluating the response to determine whether to repeat one or more of the previous process steps (214).

The system and method described herein may be used to wake a resting person, such as an off-duty pilot. The method that is implemented is effective and is done in a manner in which the resting person's cognitive abilities are not diminished.

Those of skill in the art will appreciate that the various illustrative logical blocks, modules, circuits, and algorithm steps described in connection with the embodiments disclosed herein may be implemented as electronic hardware, computer software, or combinations of both. Some of the embodiments and implementations are described above in terms of functional and/or logical block components (or modules) and various processing steps. However, it should be appreciated that such block components (or modules) may be realized by any number of hardware, software, and/or firmware components configured to perform the specified functions. To clearly illustrate this interchangeability of hardware and software, various illustrative components, blocks, modules, circuits, and steps have been described above generally in terms of their functionality. Whether such functionality is implemented as hardware or software depends upon the particular application and design constraints imposed on the overall system. Skilled artisans may implement the described functionality in varying ways for each particular application, but such implementation decisions should not be interpreted as causing a departure from the scope of the present invention. For example, an embodiment of a system or a component may employ various integrated circuit components, e.g., memory elements, digital signal processing elements, logic elements, look-up tables, or the like, which may carry out a variety of functions under the control of one or more microprocessors or other control devices. In addition, those skilled in the art will appreciate that embodiments described herein are merely exemplary implementations.

The various illustrative logical blocks, modules, and circuits described in connection with the embodiments disclosed herein may be implemented or performed with a general-purpose processor, a digital signal processor (DSP), an application specific integrated circuit (ASIC), a field programmable gate array (FPGA) or other programmable logic device, discrete gate or transistor logic, discrete hardware components, or any combination thereof designed to perform the functions described herein. A general-purpose processor may be a microprocessor, but in the alternative, the processor may be any conventional processor, controller, microcontroller, or state machine. A processor may also be implemented as a combination of computing devices, e.g., a combination of a DSP and a microprocessor, a plurality of microprocessors, one or more microprocessors in conjunction with a DSP core, or any other such configuration.

The steps of a method or algorithm described in connection with the embodiments disclosed herein may be embodied directly in hardware, in a software module executed by a processor, or in a combination of the two. A software module may reside in RAM memory, flash memory, ROM memory, EPROM memory, EEPROM memory, registers, hard disk, a removable disk, a CD-ROM, or any other form of storage medium known in the art. An exemplary storage medium is coupled to the processor such that the processor can read information from, and write information to, the storage medium. In the alternative, the storage medium may be integral to the processor. The processor and the storage medium may reside in an ASIC.

Techniques and technologies may be described herein in terms of functional and/or logical block components, and with reference to symbolic representations of operations, processing tasks, and functions that may be performed by various computing components or devices. Such operations, tasks, and functions are sometimes referred to as being computer-executed, computerized, software-implemented, or computer-implemented. In practice, one or more processor devices can carry out the described operations, tasks, and functions by manipulating electrical signals representing data bits at memory locations in the system memory, as well as other processing of signals. The memory locations where data bits are maintained are physical locations that have particular electrical, magnetic, optical, or organic properties corresponding to the data bits. It should be appreciated that the various block components shown in the figures may be realized by any number of hardware, software, and/or firmware components configured to perform the specified functions. For example, an embodiment of a system or a component may employ various integrated circuit components, e.g., memory elements, digital signal processing elements, logic elements, look-up tables, or the like, which may carry out a variety of functions under the control of one or more microprocessors or other control devices.

When implemented in software or firmware, various elements of the systems described herein are essentially the code segments or instructions that perform the various tasks. The program or code segments can be stored in a processor-readable medium or transmitted by a computer data signal embodied in a carrier wave over a transmission medium or communication path. The "computer-readable medium", "processor-readable medium", or "machine-readable medium" may include any medium that can store or transfer information. Examples of the processor-readable medium include an electronic circuit, a semiconductor memory device, a ROM, a flash memory, an erasable ROM (EROM), a floppy diskette, a CD-ROM, an optical disk, a hard disk, a fiber optic medium, a radio frequency (RF) link, or the like. The computer data signal may include any signal that can propagate over a transmission medium such as electronic network channels, optical fibers, air, electromagnetic paths, or RF links. The code segments may be downloaded via computer networks such as the Internet, an intranet, a LAN, or the like.

In this document, relational terms such as first and second, and the like may be used solely to distinguish one entity or action from another entity or action without necessarily requiring or implying any actual such relationship or order between such entities or actions. Numerical ordinals such as "first," "second," "third," etc. simply denote different singles of a plurality and do not imply any order or sequence unless specifically defined by the claim language. The sequence of the text in any of the claims does not imply that process steps must be performed in a temporal or logical order according to such sequence unless it is specifically defined by the language of the claim. The process steps may be interchanged in any order without departing from the scope of the invention as long as such an interchange does not contradict the claim language and is not logically nonsensical.

Furthermore, depending on the context, words such as "connect" or "coupled to" used in describing a relationship between different elements do not imply that a direct physical connection must be made between these elements. For example, two elements may be connected to each other physically, electronically, logically, or in any other manner, through one or more additional elements.

While at least one exemplary embodiment has been presented in the foregoing detailed description of the invention, it should be appreciated that a vast number of variations exist. It should also be appreciated that the exemplary embodiment or exemplary embodiments are only examples, and are not intended to limit the scope, applicability, or configuration of the invention in any way. Rather, the foregoing detailed description will provide those skilled in the art with a convenient road map for implementing an exemplary embodiment of the invention. It being understood that various changes may be made in the function and arrangement of elements described in an exemplary embodiment without departing from the scope of the invention as set forth in the appended claims.

## Claims

1. A system for waking a resting person, comprising:
a processing system configured to receive stimuli configuration data and sleep state data and configured, upon receipt of these data, to:
determine (i) a set of the available stimuli to use to awaken the person and (ii) a sequence of activation of the set available stimuli to use; and
selectively activate the set of available stimuli in the determined sequence of activation,
wherein:
the stimuli configuration data is indicative of all available waking stimuli available for use, and
the sleep state data is indicative of a current sleep state of the person.

2. The system of claim 1, further comprising:
one or more sensors in operable communication with the processing system and configured to supply the sleep state data to the processing system.

3. The system of claim 1, wherein the available waking stimuli include one or more of visual, aural, and vibrational stimuli.

4. The system of claim 1, wherein:
the processing system is further configured to determine an activation method for selectively activating the set of available stimuli; and
the activation method includes a gradual increase in an intensity of the activated stimuli, an abrupt activation at a first predetermined intensity, an abrupt activation at a second predetermined intensity that is greater in magnitude than the first predetermined intensity, multiple abrupt activations at a second predetermined intensity.

5. The system of claim 1, wherein the processing system is further configured, in response to the sleep state data, to monitor a response of the person to each of the set of available stimuli that is activated to determine an effectiveness thereof.

6. The system of claim 5, further comprising:
memory having user profile data stored therein that is associated with the person,
wherein the processing system is in operable communication with the memory and is further configured to selectively update the user profile data to reflect the response of the person to each set of available stimuli that is activated.

7. The system of claim 1, wherein the processing system is further coupled to receive a trigger signal and is configured, upon receipt of the trigger signal, to selectively activate the set of available stimuli in the determined sequence of activation.

8. The system of claim 7, wherein:
the processing system is further coupled to receive on-duty person state data, the on-duty person state data indicative of a cognitive state of an on-duty person who is not the person to be awakened; and
the processing system is configured, upon receipt of the on-duty person state data, to determine the cognitive state of the on-duty person and selectively generate the trigger in response to the determined cognitive state of the on-duty person.

9. A method of waking a person, comprising the steps of:
receiving, in a processing system, stimuli configuration data, the stimuli configuration data indicative of all available waking stimuli available for use;
receiving, in the processing system, sleep state data, the sleep state data indicative of a current sleep state of the person;
processing, in the processing system, the stimuli configuration data and the sleep state data to determine (i) a set of the available stimuli to use to awaken the person and (ii) a sequence of activation of the set available stimuli to use; and
selectively activating, via the processing system, the set of available stimuli in the determined sequence of activation.

10. The method of claim 9, further comprising:
supplying the sleep state data to the processing system from one or more sensors.

11. The method of claim 9, wherein the available waking stimuli include one or more of visual, aural, and vibrational stimuli.

12. The method of claim 9, further comprising:
determining, in the processing system, a method of selectively activating the set of available stimuli,
wherein the method of selectively activating the set of available stimuli includes a gradual increase in an intensity of the activated stimuli, an abrupt activation at a first predetermined intensity, an abrupt activation at a second predetermined intensity that is greater in magnitude than the first predetermined intensity, multiple abrupt activations at a second predetermined intensity.

13. The method of claim 9, further comprising:
monitoring, in the processing system, for a response of the person to each of set of available stimuli that is activated to determine an effectiveness thereof; and
selectively updating, via the processing system, user profile data associated with the person to reflect the response of the person to each of set of available stimuli that is activated.

14. The method of claim 9, further comprising:
generating, in the processing system, a trigger that causes the processing system to selectively activate the set of available stimuli in the determined sequence of activation.

15. The method of claim 14, wherein:
the trigger is generated in response to one or more of: a signal supplied from a wearable user interface disposed on a second person, a signal supplied from a second processing system, and a signal supplied from a communication device; and
the method further comprises:
receiving, in the processing system, on-duty person state data, the on-duty person state data representative of a cognitive state of an on-duty person who is not the person to be awakened;
processing, in the processing system, the on-duty person state data to determine the cognitive state of the on-duty person; and
selectively generating the trigger in response to determining the cognitive state of the on-duty person.
